(19) Europäisches Patentamt
European Patent Office
Office européen des brevets

(11) **EP 2 333 514 A1**

(12) **EUROPÄISCHE PATENTANMELDUNG**

(43) Veröffentlichungstag:
**15.06.2011 Patentblatt 2011/24**

(51) Int Cl.:
*G01N 11/14* (2006.01)  *G01N 11/16* (2006.01)
*G01N 33/48* (2006.01)

(21) Anmeldenummer: **09075526.5**

(22) Anmeldetag: **30.11.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **Berlin Heart GmbH**
**12247 Berlin (DE)**

(72) Erfinder:
• **Arndt, Andreas**
**12555 Berlin (DE)**
• **Nüsser, Peter**
**13347 Berlin (DE)**
• **Graichen, Kurt**
**13189 Berlin (DE)**

(74) Vertreter: **Golkowsky, Stefan**
**Pfenning, Meinig & Partner GbR**
**Patent- und Rechtsanwälte**
**Joachimstaler Strasse 10-12**
**10719 Berlin (DE)**

(54) **Einrichtung und Verfahren zur Messung von strömungsmechanisch wirksamen Materialparametern eines Fluids**

(57) Ein Verfahren und eine Einrichtung zur Messung von strömungsmechanisch wirksamen Parametern eines Fluids mit einer Fluidpumpe, die ein in einem Magnetlager (10, 10a, 11, 11a) gelagertes Förderelement (2) aufweist, sieht gemäß der Erfindung vor, dass mittels einer Anregungseinrichtung (16, 44) das Förderelement (2) der Fluidpumpe zu einer Schwingung angeregt wird, wobei die Schwingungsparameter sowie gegebenenfalls das Ausschwingverhalten gemessen und hieraus Parameter des Fluids bestimmt werden.

Fig. 4

EP 2 333 514 A1

# Beschreibung

Die Erfindung liegt auf dem Gebiet der Strömungsmechanik, insbesondere der strömungstechnischen Messung von Materialeigenschaften von Fluiden.

Für die Beurteilung und Auslegung von strömungsmechanischen Einrichtungen ist einerseits die Kenntnis von Strömungswiderständen in fluiddurchflossenen Kanälen wichtig, andererseits jedoch ebenso die genaue Kenntnis der Materialeigenschaften des strömenden Fluids, also beispielsweise der Flüssigkeit oder des Gases, das durch die Kanäle gefördert wird. Die entsprechenden Eigenschaften sind grundsätzlich dem Material eigen, jedoch im Einzelnen noch von verschiedenen Randbedingungen wie beispielsweise der Temperatur oder auch der Strömungsgeschwindigkeit (beispielsweise bei nicht newtonschen Flüssigkeiten) abhängig. Besonders bei Anwendungen in der Medizintechnik, wenn biologisch wirksame und auch biologischen Prozessen unterworfene Flüssigkeiten gefördert werden sollen, können sich die entsprechenden Eigenschaften dieser Flüssigkeiten laufend ändern. Eine besondere Anwendung dieser Art stellt die Förderung von körpereigenem Blut dar, das in seiner Viskosität von verschiedenen physiologischen Prozessen abhängig ist. Dementsprechend kann beispielsweise eine Pumpleistung bei einer Pumpe, die zur Förderung von Blut eingesetzt wird, an eine stetig überwachte Blutviskosität angepasst werden.

Aus dem Stand der Technik sind verschiedenste Methoden zur Messung der Dichte und/oder der Viskosität von Fluiden bekannt.

Entsprechende Viskosimeter sind beispielsweise als Auslaufbecher, Fallkörperviskosimeter oder Messrührantriebe aus der Literatur und auch aus der Standardisierung bekannt.

Ebenfalls bekannt sind Viskositäts- und Dichtemessgeräte, die die Wirkung eines Fluids auf ein in diesem befindliches schwingendes Element verarbeiten.

Zudem ist aus der US-Patentschrift 6581476 B1 eine Messmethode bekannt, bei der der Rotor eines Synchronmotors innerhalb einer Flüssigkeit angetrieben und gleichzeitig Drehzahl und Energieverbrauch zur Bestimmung der Viskosität gemessen werden.

Der vorliegenden Erfindung liegt vor dem Hintergrund des Standes der Technik die Aufgabe zugrunde, eine Einrichtung und ein Verfahren zu schaffen, mittels deren in möglichst wenig aufwendiger, jedoch zuverlässiger und genauer Weise strömungsmechanische Eigenschaften eines Fluids gemessen werden können.

Die Aufgabe wird gemäß der Erfindung mit einer Einrichtung mit den Merkmalen des Patentanspruchs 1 sowie mittels eines Verfahrens mit den Merkmalen des Patentanspruchs 10 gelöst.

Gemäß der Erfindung wird dabei eine Fluidpumpe verwendet, die ein in einem Magnetlager gelagertes Förderelement aufweist. Zudem weist die Einrichtung zur Messung der Materialparameter eine Anregungseinrichtung zur Schwingungsanregung des Förderelementes gegen eine durch das Magnetlager aufgebrachte Gegenkraft sowie eine Sensoreinrichtung zur Messung des Schwingungsverhaltens des Förderelementes auf.

Die erfindungsgemäße Einrichtung erlaubt somit unter Einsatz einer üblicherweise zur Förderung des Fluids ohnehin vorhandenen Fluidpumpe die Messung der interessierenden strömungsmechanisch wirksamen Materialparameter, ohne dass ein gesondertes Element in den Strömungspfad eingepasst werden müsste oder in aufwendiger Weise Proben entnommen werden müssten. Es ist lediglich eine Anregungseinrichtung für das Förderelement und eine Sensoreinrichtung zur Erfassung des Schwingungsverhaltens notwendig. Dabei ist ein typisches Förderelement durch einen Rotor gebildet, der je nach Bauart der Pumpe in axialer oder radialer Richtung das Fluid fördert und entsprechend in einem Magnetlager reibungsarm gelagert sein kann. Ein derartiges Magnetlager kann entsprechend als Radiallager oder Axiallager ausgebildet sein. Eine aktive Regelung des Lagers ist vorgesehen, um die Position des Förderelementes zu stabilisieren und Rückwirkungen des zu fördernden Fluids bei Einsetzen der Pumpleistung auf das Lager auszugleichen. Eine derartige Magnetlagerregelung sieht üblicherweise einen Positionssensor für das Förderelement sowie eine Steuereinrichtung zur Steuerung zusätzlich erzeugter Magnetkräfte beispielsweise durch einen Elektromagneten vor. Die Steuereinrichtung kann dazu beispielsweise den Strom durch eine magnetfelderzeugende Spule steuern. Der entsprechende Positionssensor kann ebenfalls die Messung der Position des Förderelementes durch eine sensitive Magnetspule durchführen.

Wenn eine aktive Lagerregelung in der Richtung des Magnetlagers vorgesehen ist, in der auch die Schwingung stattfindet, muss sichergestellt werden, dass die Regelung mit der Schwingung nicht unkontrolliert wechselwirkt. Dies kann beispielsweise dadurch geschehen, dass die Regelung eine andere Zeitkonstante aufweist als die Schwingung, beispielsweise wesentlich höherfrequent oder mit wesentlich geringerer Frequenz als die Schwingung arbeitet.

Es kann auch vorgesehen sein, dass die Lagerregelung mit der Schwingung interagiert, wobei dann bei der Auswertung der Schwingung die veränderlichen Lagerkräfte berücksichtigt werden müssen.

Die erfindungsgemäße Einrichtung weist zur Analyse der Schwingung in der Sensoreinrichtung vorteilhaft einen ersten Sensor zur Messung einer Schwingungsfrequenz des Förderelementes und/oder einen zweiten Sensor zur Messung der Schwingungsamplitude des Förderelementes sowie gegebenenfalls eine Zeiterfassungseinrichtung zur Messung des Ein- und/oder Ausschwingverhaltens des Förderelementes auf.

Sowohl die Eigenfrequenz nach Beendigung der Anregung als auch die Schwingungsamplitude bzw. das Ein- oder Ausschwingverhalten des freien Schwingers weisen eine substantielle Abhängigkeit von der Dichte und/oder der Zähigkeit des Fluides auf, das das Förderelement umgibt. Zudem spielen bei der Messung dieser Größen die geometrischen Verhältnisse innerhalb der Fluidpumpe eine Rolle, wenn beispielsweise durch die Schwingung in Spalten das Fluid periodisch verdrängt wird. Somit ist zur Bestimmung der strömungsmechanischen Eigenschaften des Fluids durch die beschriebene Messung eine Kalibrierung notwendig.

Der physikalisch relevante Zusammenhang zwischen den gesuchten, strömungsmechanisch wirksamen Materialparametern (Stoffwerten)

$\rho$     Dichte des Fluids in kg/m$^3$
$\eta$     dynamische Zähigkeit des Fluids in Pas

und messtechnisch erfassbaren Größen beim Schwingen des magnetisch gelagerten Rotors nach geeigneter Schwingungsanregung lässt sich vereinfacht auf folgende Weise beschreiben: Für den in Richtung der Drehachse des Rotors, der x-Achse, schwingenden Rotor gilt eine Bewegungsgleichung der Form

$$m_{gesamt} * \frac{d^2 x}{dt^2} + C_V * \frac{dx}{dt} + k * x = 0$$

mit der Gesamtmasse m$_{gesamt}$, dem Reibungskoeffizienten C$_V$ und der Steifigkeit k des Lagers in x-Richtung.

Es ist bekannt, dass beim Schwingen eines Körpers in einem ihn umgebenden Fluid nicht nur der Körper selbst, sondern auch ein bestimmter Anteil des benachbarten Fluids beschleunigt werden muss. Die Gesamtmasse m$_{gesamt}$ umfasst deshalb nicht nur die bekannte Rotormasse, sondern auch eine "Zusatzmasse", deren Größe von der Geometrie der Anordnung und der Dichte des umgebenden Fluids abhängt. Die Größe des "Geometrie-Faktors" dieser "Zusatzmasse" ist für viele Anordnungen bekannt.

Das in der obigen Schwingungsgleichung beschriebene Kräftegleichgewicht wird weiterhin durch geschwindigkeitsproportionale Reibungskräfte bestimmt. Der Faktor C$_V$ ist der gesuchten dynamischen Zähigkeit des Fluids proportional.

In der Schwingungsgleichung verbergen sich demnach die gesuchten Materialkennwerte (Stoffwerte) Dichte und dynamische Zähigkeit, die mittels einer Analyse des Schwingungsverhaltens des Rotors auf der Grundlage geeigneter Kalibrierungen ermittelt werden können.

Grundsätzlich kann zudem bei der erfindungsgemäßen Einrichtung vorgesehen sein, dass die Anregungseinrichtung mit einer Einrichtung zur Regelung einer magnetischen Lagerkraft verbunden ist.

Damit kann beispielsweise durch Aufbringen eines Stroms auf eine Magnetspule nicht nur die magnetische Lagerkraft geregelt, sondern auch ein Stoßimpuls auf ein Förderelement zur Anregung einer Schwingung gegeben werden.

Auch der erste und/oder zweite Sensor kann mit einem Positionssensor der Magnetlagerung der Förderelementes, also beispielsweise einer sensorischen Magnetspule verbunden, baulich vereinigt oder sogar identisch sein.

Die Erfindung bezieht sich außer auf eine Einrichtung auch auf ein Verfahren zur Messung von strömungsmechanisch wirksamen Materialparametern gemäß Patentanspruch 10.

Bei diesem Verfahren wird mittels einer Anregungseinrichtung eine Schwingung auf ein Förderelement einer Fluidpumpe gegeben, das in einem Magnetlager gelagert ist, und es wird das Schwingungsverhalten des Förderelementes gemessen.

Vorteilhaft werden dabei die Schwingungsfrequenz des Förderelementes, dessen Amplitude oder eine Abklingzeit der Schwingung oder auch der Energieaufwand bei der Schwingungsanregung gemessen.

Dies kann besonders genau bei ruhendem Förderelement durchgeführt werden. Es kann jedoch auch vorteilhaft sein, die Messung während der Fluidförderung durchzuführen, um Unterbrechungen der Fluidförderung zu vermeiden und dennoch die Materialeigenschaften laufend überwachen zu können. Dies ist insbesondere bei der Förderung von biologisch wirksamen Flüssigkeiten, insbesondere in lebenden Körpern vorteilhaft, um die entsprechenden von der Versorgung mit dem Fluid abhängigen Prozesse nicht zu stören.

Letztlich bezieht sich die Erfindung auch auf eine Verwendung einer Fluidpumpe zur Durchführung eines Messverfahrens der beschriebenen Art.

Im Folgenden wird die Erfindung anhand eines Ausführungsbeispiels in einer Zeichnung gezeigt und nachfolgend beschrieben.

Dabei zeigt

Fig. 1     in einem Längsschnitt schematisch eine Axialpumpe mit geregeltem magnetischem Axiallager,

Fig. 2     schematisch die Funktionsweise der magnetischen Lagerregelung und des Pumpenantriebs,

Fig. 3     schematisch die Messung der Reaktionskraft auf das Axiallager der Pumpe im Förderbetrieb, die Messung der Drehzahl und hieraus resultierend bei bekannten Fluideigenschaften die Ableitung von strömungsmechanischen Eigenschaften eines fluiddurchströmten, von der Pumpe verschiedenen Bauteils sowie

Fig. 4     schematisch die Funktion der Schwingungsanregung bei einer Axialpumpe und die Messung der Schwingung und

Fig. 5     ein Messdiagramm des Schwingungsverhaltens.

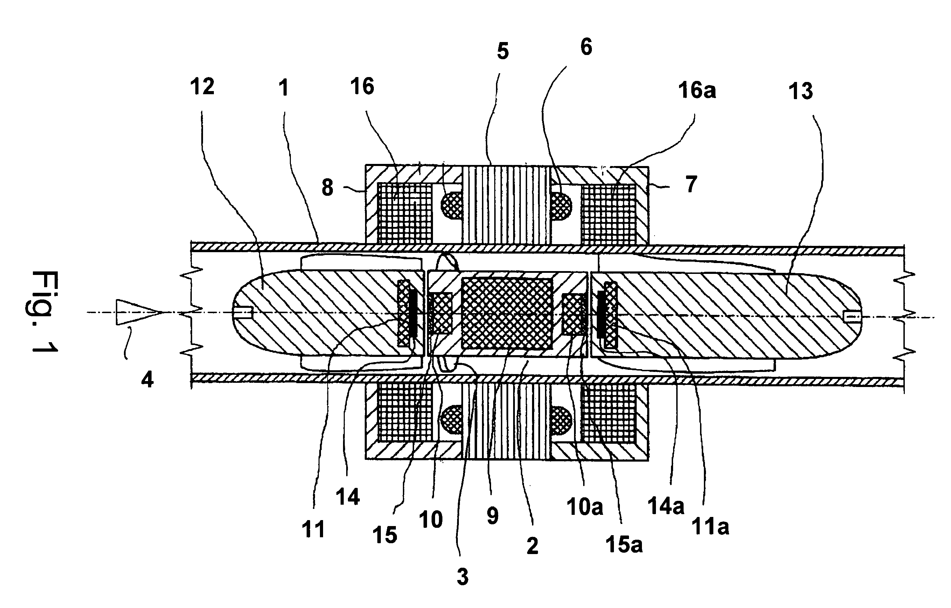

Fig. 1 zeigt schematisch in einem Längsschnitt eine magnetgelagerte Axialpumpe, wie sie beispielsweise als Blutpumpe für den menschlichen Körper Anwendung findet. Die Pumpe ist in ein zylindrisches Rohr 1 eingesetzt und weist einen Rotor 2 mit Rotorblättern 3 zum Vortrieb eines Fluids in Strömungsrichtung 4 auf.

Der Antrieb für den Rotor 2 sieht ein Blechpaket 5, Wicklungen 6 sowie Jochteile 7, 8 vor, die gemeinsam mit dem Blechpaket 5 einen hochpermeablen Magnetkreis bilden, der über Permanentmagneten 9 im Kern des Rotors 5 geschlossen wird, so dass insgesamt ein Synchronmotor oder auch ein bürstenloser Gleichstrommotor mit außen liegendem Stator gebildet ist, der elektronisch kommutiert wird.

Der Rotor 2 ist mittels Permanentmagneten 10, 10a, deren Magnetachse parallel zur Flussrichtung 4 ausgerichtet ist, sowie ortsfesten Permanentmagneten 11, 11a in axialer Richtung magnetisch berührungsfrei gelagert.

Die Permanentmagnete 10, 11 und 10a, 11a sind jeweils so ausgerichtet, dass sie sich paarweise anziehen. Damit ist in axialer Richtung bestenfalls ein labiles Gleichgewicht gebildet, so dass zur berührungsfreien Lagerung zusätzlich eine Regeleinrichtung vorgesehen werden muss.

Zur Erfassung der axialen Position des Rotors 2 ist jeweils in dem ortsfesten, in Verlängerung des Rotors innerhalb des Rohres 1 liegenden Teil der Axialpumpe 12, 13 je eine Sensorspule 14, 14a vorgesehen, der innerhalb des Rotors jeweils ein Kurzschlussring 15, 15a gegenüberliegt, so dass mittels der Induktivität der Sensorspulen 14, 14a jeweils der Abstand zum jeweiligen Kurzschlussring 15, 15a und damit die Position des Rotors 2 gemessen werden kann.

Die entsprechenden Messgrößen werden als Ausgangsgröße einer Regeleinrichtung zugeleitet, die in Abhängigkeit von der gemessenen Position bzw. von der Differenz zur Sollposition des Rotors einen Strom durch zwei Steuerspulen 16, 16a steuert, die die jeweiligen Axialfelder der Permanentmagneten 11, 11a stärken oder schwächen, um die Anziehungskraft der Permanentmagneten 10, 11, 10a, 11a derart zu steuern, dass der Rotor 5 in axialer Richtung eine Sollposition einnimmt.

Wird nun der Rotor durch Einschalten des Antriebs derart in Drehung versetzt, dass ein in dem Rohr 1 befindliches Fluid in der Richtung 4 befördert wird, so ergibt sich eine Reaktionskraft, die der auf das Fluid in der Richtung 4 wirkenden Kraft entgegengesetzt ist und den Rotor axial auslenkt.

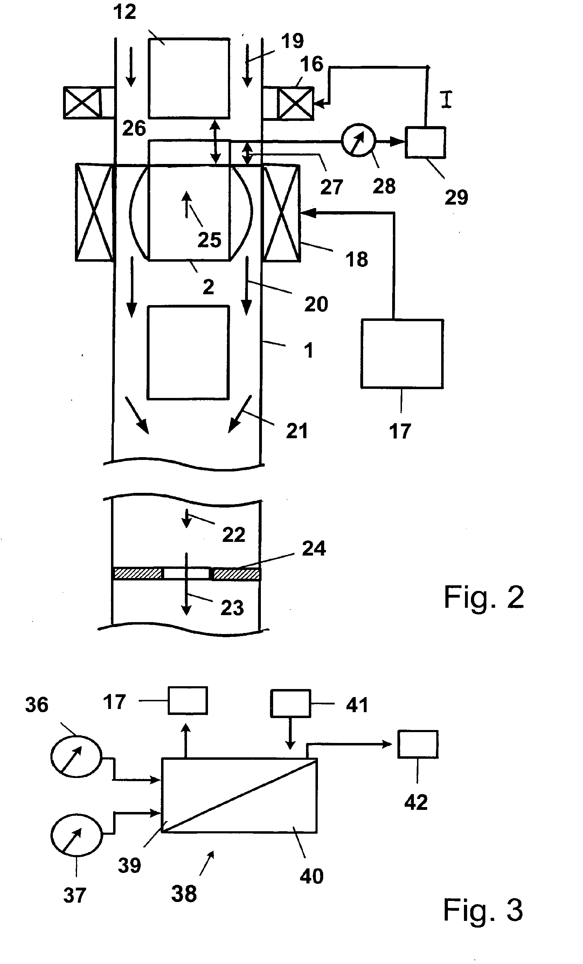

Dieser Vorgang ist näher anhand der Fig. 2 beschrieben, in der die in der Fig. 1 bereits gezeigten Elemente bei Übereinstimmung mit denselben Bezugszeichen bezeichnet sind.

Die Antriebssteuerung ist in der Fig. 2 mit 17 bezeichnet und wirkt auf eine in der Fig. 2 nur schematisch dargestellte Antriebswicklung 18, die den Rotor 2 in Rotation versetzt. Handelt es sich wie beschrieben um einen Synchronmotor, so ist mit der Antriebssteuerung 17 bereits eine Drehzahl vorgebbar. Anderenfalls kann im Bereich des Rotors zusätzlich ein Drehzahlsensor zur Messung der Drehzahl vorgesehen werden.

Wenn der Rotor in Rotation versetzt ist, fließt das Fluid, wie dies in der Fig. 2 mit den Pfeilen 19, 20, 21, 22 und 23 dargestellt ist, durch das Rohr 1 bzw. seine Fortsetzung. Die Strömungswiderstandsparameter des durchströmten Elementes 24 in Form einer Lochblende können im Betrieb durch Erfassung von Meßgrößen im Bereich der Axialpumpe ermittelt werden.

Beim Betrieb des Pumpenantriebs wie oben dargestellt ergibt sich eine Kraft auf den Rotor, die in Richtung des Pfeils 25 gerichtet ist und den axialen Spalt zu dem ortsfesten Bauteil 12 zu verkleinern sucht. Die Größe dieses Spaltes ist in der Fig. 2 mit dem Bezugszeichen 26 bezeichnet, während die Abweichung von der Sollposition des Rotors mit 27 bezeichnet ist. Diese Abweichung wird mittels des Sensors 28 erfasst, der diese Größe an die Steuer-/Regeleinrichtung 29 für die Axialposition des Rotors weitergibt. Diese Steuer-/Regeleinrichtung bestimmt entsprechend den notwendigen Strom i durch die Steuerspule 16, die das Axialmagnetfeld beeinflusst, welches auf wenigstens einen der Permanentmagneten in dem Rotor 2 wirkt. Auf diese Weise wird die Axialposition des Rotors geregelt und stabil gehalten.

Die zur Aussteuerung notwendige Stromstärke i ist ein Maß für die auf den Rotor 2 wirkende Gegenkraft bzw. die Pumpenlast oder auch den durch die Pumpe erzeugten Druckunterschied in dem geförderten Fluid.

Anhand der Fig. 3 soll im Folgenden beispielhaft die Struktur der Messeinrichtung und ihre Anwendung zur Vermessung des Strömungspfades dargestellt werden.

Dabei ist mit 36 der Sensor für die Stromstärke bezeichnet, die für die axiale Lagerstabilisierung notwendig ist, 37 bezeichnet einen Drehzahlsensor des Rotors 2. Die Sensoren sind mit der Auswerteeinrichtung 38 verbunden, die eine Vergleichseinrichtung 39 sowie eine Speichereinrichtung 40 aufweist. In der Speichereinrichtung 40 sind die entsprechenden Kennfelder für Drehzahlen und Stromwerte hinterlegt. Die Auswerteeinrichtung 38 steuert zudem den Antrieb 17 des Rotors an. Außerdem kann die Auswerteeinrichtung über die Eingabeeinheit 41 Informationen über die Art des verwendeten Fluids erhalten.

Im Anschluss an den Vergleichsvorgang gibt die Auswerteeinrichtung 38 den ermittelten Strömungswiderstandsparameter an eine Ausgabeeinrichtung 42.

Die Auswertung kann auch anstelle des Vergleichs von Messwerten mit einem Kennfeld durch Berechnung anhand eines Auswertungsalgorithmus erfolgen.

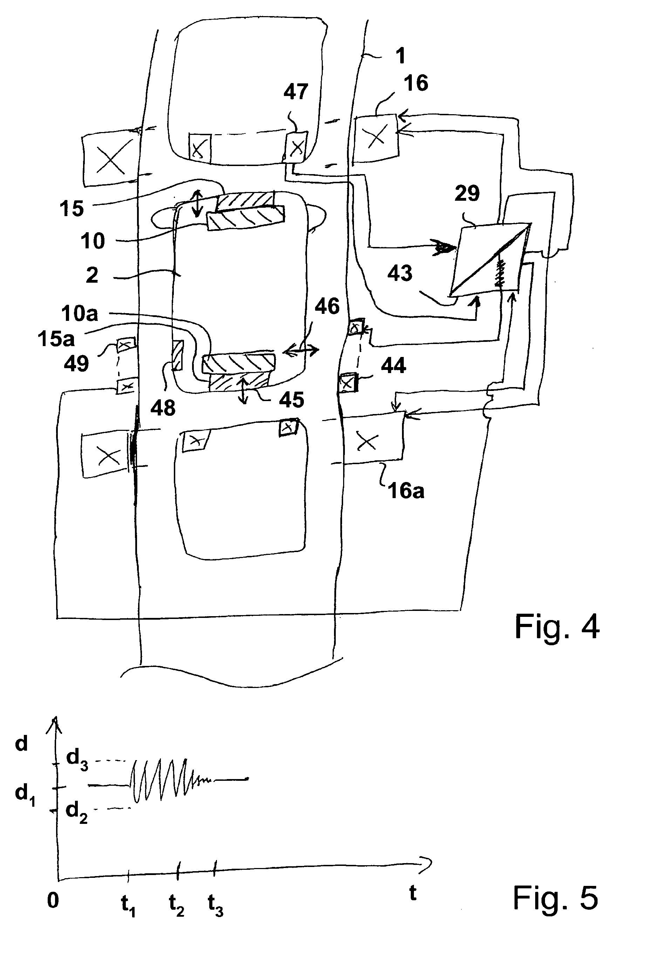

Anhand von Fig. 4 soll im Folgenden dargestellt werden, wie eine ähnliche Einrichtung im Rahmen einer Vermessung von Materialeigenschaften genutzt werden kann. Fig. 4 zeigt dazu in einem Rohr 1 schematisch im Längsschnitt einen Rotor 2, der magnetisch mittels zweier ringförmiger Permanentmagnete 10, 10a axial gelagert ist, die im Axialfeld einer nicht näher dargestellten ein Axialfeld erzeugenden stationären Magneteinrichtung liegen, wobei die steuerbaren Magnetspulen 16, 16a zur Axialstabilisierung des Rotors 2 dienen und durch die Regeleinrichtung 29 ansteuerbar sind. Die Spulen 16, 16a erzeugen dabei ein zusätzliches axiales Magnetfeld zur Positionierung des Rotors 2 in Axialrichtung.

Mit der Regeleinrichtung 29 verbunden ist eine Anregungseinrichtung 43 zur Schwingungsanregung in axialer oder radialer Richtung, die zur Schwingungsanregung in axialer Richtung beispielsweise eine der Spulen 16, 16a ansteuert oder zur Schwingungsanregung in radialer Richtung eine zusätzliche, außen an dem Rohr 1 angeordnete Anregungsspule 44.

Eine Schwingungsanregung in axialer Richtung hat eine axiale Schwingung des Rotors 2 in der durch den Pfeil 45 angedeuteten axialen Richtung zur Folge, während eine radiale Schwingungsanregung eine Schwingung in radialer Richtung, angedeutet durch den Pfeil 46, zur Folge hat.

Das Schwingungsverhalten kann einerseits im Fall der Axialschwingung durch eine Sensorspule 47 aufgenommen werden, in deren Feldbereich ein Tauchkörper 15 des Rotors einwirkt. Diese Sensorspule 47 kann zusätzlich auch zur Positionsdetektion des Rotors 2 allgemein für die Lagerregelung dienen und ist mit der Regeleinrichtung 29 in diesem Fall ebenso wie mit der Messeinrichtung 43 zur Detektion des Schwingungsverhaltens verbunden.

Im Fall einer radialen Schwingung, die durch die Anregungsspule 44 initiiert werden kann, kann die radiale Position des Rotors 2 mittels eines Tauchkörpers 48 und einer Sensorspule 49 außen an dem Rohr 1 detektiert und ebenfalls der Messeinrichtung 43 zugeführt werden.

Alternativ zu der in der Fig. 4 dargestellten Axialanregungsweise kann auch zum Aufbringen eines Anregungssignals eine gesonderte magnetisch wirksame Spule vorgesehen sein, die von der für die Lagerregelung verwendeten Spule verschieden ist.

Ebenso kann die Sensorspule, die für das Aufnehmen des Schwingungsverhaltens verwendet wird, von der Sensorspule zur Detektion der Axialposition des Rotors für die Lagerregelung verschieden sein.

Beim Aufbringen einer Radialschwingung stellt sich das Problem der Interaktion mit der Lagerregelung nicht, solange die Magnetkonstellation der im Rohr befindlichen Statoren sowie des Rotors 2 derart gestaltet ist, dass die Magnetlagerung in radialer Richtung selbstzentrierend ist. Dies kann beispielsweise dadurch der Fall sein, dass die Statoren jeweils kreisscheibenförmige Magnete an ihren Stirnseiten tragen, die ein axiales Magnetfeld erzeugen und die jeweils Magneten 10, 10a in dem Rotor 2 gegenüberstehen, welche ebenfalls mit ihrem Magnetfeld axial ausgerichtet und konzentrisch zu diesen angeordnet sind. In diesem Fall ist das Lager radial selbstzentrierend, so dass die Rotorposition sich nach Aufbringen eines Auslenkungsimpulses in radialer Richtung von selbst nach Durchlaufen einer Schwingung mit entsprechender Dämpfung wieder zentrieren wird. In der Fig. 5 ist das typische Schwingungsverhalten in Form der Amplitude, aufgetragen auf der Ordinate, gegenüber der Zeit, aufgetragen auf der Abszisse, dargestellt. Zwischen dem Zeitpunkt 0 und $t_1$ nimmt der Rotor eine Position $d_1$ ein, die beispielsweise einen stabilen Zustand zwischen dem Rotor und einem Stator darstellt.

Zum Zeitpunkt $t_1$ wird eine periodische Auslenkung, beispielsweise in axialer Richtung, aufgebracht und für eine Zeitlang bis zum Zeitpunkt $t_2$ als erzwungene Schwingung aufrechterhalten. Es ergibt sich in Abhängigkeit von der eingebrachten Leistung eine bestimmte Amplitude der Schwingung, $d_3 - d_2$.

Zum Zeitpunkt $t_2$ wird die Schwingungsanregung ausgeschaltet, und die Schwingung klingt näherungsweise exponentiell ab (s. logarithmisches Dekrement), wobei zum Zeitpunkt $t_3$ praktisch keine oder nur eine definiert reduzierte Auslenkung feststellbar ist. Die Zeitkonstante des Abklingverhaltens zwischen $t_2$ und $t_3$ ist messbar und beschreibt die Dissipation der Schwingungsenergie, die ebenso wie die Amplitude von der Zähigkeit des den Rotor umgebenden Fluids abhängig ist. Im Übrigen befindet sich der Rotor zwischen $t_2$ und $t_3$ in einem Schwingungszustand der freien Schwingung, in dem auch die Frequenz der Schwingung bzw. die Differenz zur Eigenfrequenz von dem den Rotor umgebenden Fluid abhängt und damit einen Rückschluss auf Stoffparameter des Fluids zulässt.

Die beschriebenen Größen des Schwingungsverhaltens erlauben damit eine Bestimmung von strömungstechnisch wirksamen Parametern des Fluids, insbesondere der Flüssigkeit, die den Rotor umgibt.

Damit erlaubt die Erfindung mit einfachsten Mitteln die Verwendung einer oft ohnehin vorhandenen Fluidpumpe zur Messung von strömungstechnischen Eigenschaften des geförderten Fluids und gegebenenfalls in Verbindung damit durch die Messung des Druckgefälles in der Pumpe und der Drehzahl ebenfalls Rückschluss auf zusätzliche von dem Fluid durchströmte Elemente.

## Patentansprüche

1.  Einrichtung zur Messung von strömungsmechanisch wirksamen Materialparametern eines Fluids, insbesondere einer Flüssigkeit, mit einer Fluidpumpe, die ein in einem Magnetlager (10, 10a, 11, 11a, 16, 16a) gelagertes Förderelement (2) aufweist, mit einer Anregungseinrichtung (16, 44) zur Schwingungsanregung des Förderelementes gegen eine durch das Magnetlager aufgebrachte Gegenkraft und mit einer Sensoreinrichtung (47, 49) zur Messung des Schwingungsverhaltens des Förderelementes.

2.  Einrichtung nach Anspruch 1, **dadurch gekenn-**

**zeichnet, dass** die Sensoreinrichtung (47, 49) einen ersten Sensor zur Messung einer Schwingungsfrequenz des Förderelementes aufweist.

3. Einrichtung nach Anspruch 1 oder 2, **dadurch gekennzeichnet, dass** die Sensoreinrichtung (47, 49) einen zweiten Sensor zur Messung der Schwingungsamplitude des Förderelementes (2) aufweist.

4. Einrichtung nach Anspruch 1, 2 oder 3, **dadurch gekennzeichnet, dass** die Sensoreinrichtung (47, 49) eine Zeiterfassungseinrichtung zur Messung des Ein- und/oder Ausschwingverhaltens des Förderelementes (2) aufweist.

5. Einrichtung gemäß einem der vorangehenden Ansprüche, **dadurch gekennzeichnet, dass** die Anregungseinrichtung (16) mit einer Einrichtung zur Regelung einer magnetischen Lagerkraft verbunden ist.

6. Einrichtung gemäß einem der Ansprüche 2, 3, 4 oder 5, soweit auf 2 oder 3 zurückbezogen, **dadurch gekennzeichnet, dass** der erste Sensor und/oder der zweite Sensor mit einem Positionssensor (47) einer Magnetlagerung des Förderelementes (2) verbunden ist.

7. Einrichtung nach einem der vorangehenden Ansprüche, **dadurch gekennzeichnet, dass** die Fluidpumpe als Axialpumpe und das Förderelement (2) als Rotor ausgebildet ist.

8. Einrichtung nach Anspruch 7, **dadurch gekennzeichnet, dass** das Magnetlager (10, 10a, 11, 11a, 16, 16a) als Axiallager ausgebildet ist.

9. Einrichtung nach einem der Ansprüche 1 bis 6, **dadurch gekennzeichnet, dass** das Magnetlager als Radiallager ausgebildet ist.

10. Verfahren zur Messung von strömungsmechanisch wirksamen Materialparametern eines Fluids, insbesondere einer Flüssigkeit, mittels einer Fluidpumpe, die ein in einem Magnetlager (10, 10a, 11, 11a, 16, 16a) gelagertes Förderelement (2) aufweist, wobei mittels einer Anregungseinrichtung (16, 44) eine Schwingung des Förderelementes gegen eine durch das Magnetlager aufgebrachte Gegenkraft angeregt und das Schwingungsverhalten des Förderelementes gemessen wird.

11. Verfahren nach Anspruch 10, **dadurch gekennzeichnet, dass** nach dem Ende der Anregung die Frequenz der Schwingung des Förderelementes (2) gemessen wird.

12. Verfahren nach Anspruch 10 oder 11, **dadurch ge-**

**kennzeichnet, dass** die Amplitude der Schwingung gemessen wird.

13. Verfahren nach Anspruch 10, 11 oder 12, **dadurch gekennzeichnet, dass** eine Abklingzeit der Schwingung nach der Anregung gemessen wird.

14. Verfahren nach Anspruch 10, 11, 12 oder 13, **dadurch gekennzeichnet, dass** der Energieaufwand für die Schwingungsanregung gemessen wird.

15. Verfahren nach einem der Ansprüche 10 bis 14, **dadurch gekennzeichnet, dass** die Messung bei ruhendem, nicht rotierendem Förderelement (2) durchgeführt wird.

16. Verfahren nach einem der Ansprüche 10 bis 14, **dadurch gekennzeichnet, dass** die Messung während der Fluidförderung durchgeführt wird.

17. Verfahren nach einem der Ansprüche 10 bis 16, **dadurch gekennzeichnet, dass** im Betrieb der Fluidpumpe die Drehzahl des Förderelementes (2) und/oder eine Rückwirkungskraft des erzeugten Fluiddrucks auf ein Magnetlager des Förderelements gemessen wird.

18. Verwendung einer Fluidpumpe mit einem in einem Magnetlager gelagerten Förderelement für ein Messverfahren gemäß einem der Patentansprüche 10 bis 17.

Fig. 1

Fig. 2

Fig. 3

Fig. 4

Fig. 5

Europäisches
Patentamt

European
Patent Office

Office européen
des brevets

## EUROPÄISCHER RECHERCHENBERICHT

Nummer der Anmeldung

EP 09 07 5526

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 967 475 A1 (SULZER ELECTRONICS AG [CH]; LUST ANTRIEBSTECHNIK GMBH [DE] LEVITRONIX) 29. Dezember 1999 (1999-12-29) * Absatz [0004] *<br>----- | 1-18 | INV.<br>G01N11/14<br>G01N11/16<br><br>ADD.<br>G01N33/48 |
| X | EP 0 971 212 A1 (SULZER ELECTRONICS AG [CH]; LUST ANTRIEBSTECHNIK GMBH [DE]) 12. Januar 2000 (2000-01-12) * Absatz [0027] - Absatz [0029] *<br>----- | 1-18 | |
| A | DE 196 13 388 A1 (NTN TOYO BEARING CO LTD [JP]) 10. Oktober 1996 (1996-10-10) * Spalte 6, Zeile 5 - Spalte 6, Zeile 29; Abbildung 6 *<br>----- | 1-18 | |

| | RECHERCHIERTE SACHGEBIETE (IPC) |
|---|---|
| | G01N |

Der vorliegende Recherchenbericht wurde für alle Patentansprüche erstellt

| Recherchenort | Abschlußdatum der Recherche | Prüfer |
|---|---|---|
| München | 25. November 2010 | Thomte, Peter |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 07 5526

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-11-2010

| Im Recherchenbericht angeführtes Patentdokument | | Datum der Veröffentlichung | Mitglied(er) der Patentfamilie | | Datum der Veröffentlichung |
|---|---|---|---|---|---|
| EP 0967475 | A1 | 29-12-1999 | US | 6711943 B1 | 30-03-2004 |
| EP 0971212 | A1 | 12-01-2000 | KEINE | | |
| DE 19613388 | A1 | 10-10-1996 | US | 5725357 A | 10-03-1998 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6581476 B1